# EUROPEAN PATENT APPLICATION

(11) **EP 3 858 342 A1**
(43) Date of publication of application: **04.08.2021**
(21) Application number: 19813064.3
(22) Date of filing: 27.09.2019
(51) Int. Cl.: A61K 31/175, A61K 31/341, A61K 31/381, A61K 31/404, A61K 45/06, A61P 25/16, A61P 25/28, A61P 43/00, C07C 245/00, C07D 307/00, C07D 333/00

(54) **ACYLHYDRAZONES FOR THE TREATMENT OF NEUROLOGICAL DISEASES**

(30) Priority: 27.09.2018 ES 201830933; 02.04.2019 EP 19382242
(71) Applicant: Consejo Superior de Investigaciones Científicas (CSIC), 28006 Madrid (ES); Fundación Para La Investigación Biomédica Del Hospital Universitario Ramón Y Cajal, 28034 Madrid (ES)
(72) Inventor: PÉREZ FERNÁNDEZ, Ruth, 28040 Madrid (ES); CANAL MARTÍN, Andrea, 28040 Madrid (ES); SANCHEZ BARRENA, María José, 28006 Madrid (ES); MANSILLA APARICIO, Alicia, 28034 Madrid (ES)
(74) Representative: Hoffmann Eitle
(86) International application number: PCT/ES2019/070649
(87) International publication number: WO 2020/065119

(57) **Abstract**

The present invention relates to a group of compounds with an acylhydrazone structural core having the capacity to modulate interaction between NCS-1 and Ric8a proteins involved in the process of regulating the number of synapses and the neurotransmitter release probability. These compounds are therefore useful for the treatment of neurological diseases in which the number of synapses is affected, such as Alzheimer's disease, Huntington's disease, or Parkinson's disease.

## Description

The present invention relates to a group of compounds with an acylhydrazone structural core exhibiting activity in modulating interaction between NCS-1 and Ric8a proteins involved in the process of regulating the number of synapses and the neurotransmitter release probability. Due to this, the compounds of the invention are useful for the treatment of neurological diseases in which the number of synapses is affected, such as Alzheimer's disease, Huntington's disease, or Parkinson's disease.

### BACKGROUND OF THE INVENTION

In order to transmit nerve impulses intended for coordinating all bodily functions, neurons communicate by means of synapses. These require the formation of a neurotransmitter release locus in the presynaptic neuron and an associated receiving field in the postsynaptic neuron. For proper nerve transmission functioning, a suitable balance must be struck between the number of release sites and neurotransmitter release probability. At times, this balance becomes imbalanced, negatively affecting the functioning of the nervous system. When there are defects in nervous system development, an imbalance occurs due to an excess of synapses causing disorders such as Angelman syndrome, fragile X chromosome syndrome, Rett syndrome, other autism spectrum disorders, epilepsy, or bipolar disorder, among others. Other times, the problem is synapse loss which results in the development of neurodegenerative diseases such as Alzheimer's disease, Huntington's disease, Parkinson's disease, etc., which are increasingly more prevalent as a result of population aging.

Synapse restoration by means of drugs has high therapeutic value. It has been demonstrated that by inhibiting interaction between neuronal calcium sensor 1 (NCS-1) and guanine exchange factor (Ric8a) proteins, the normal number of synapses in an animal model of fragile X syndrome is restored (Mansilla, A. et. al. PNAS 2017, 114, E999-E1008). However, there are no publications today disclosing compounds which stabilize the interaction of the protein complex (NCS-1 :Ric8a) and re-establish lost synapses, which would contribute to an improvement in cognitive functions and memory which are impaired in neurodegenerative diseases such as Alzheimer's disease.

Compounds with an acylhydrazone structural core that are useful for the treatment of neurological diseases such as epilepsy (Angelova et al., J. Drug Dev Res. 2016 Nov, 77(7):379-392) or Alzheimer's disease (Dias Viegas FP et al. Eur J Med Chem. 2018 Mar 10, 147:48-65) have been described, although compounds of this type which effectively treat neurological diseases by improving the number of synapses by modulating NCS-1 and Ric8a protein interaction have not been described.

### DESCRIPTION OF THE INVENTION

The protein complex formed by NCS-1 and Ric8 regulates the number of synapses and neurotransmitter release probability, so it is considered a good therapeutic target for the treatment of diseases in which the synapse is impaired. Based on fluorescence spectroscopy studies, the affinity of compounds for *d*NCS-1 (neuronal calcium sensor of *Drosophila*) has been proven. Neuronal calcium sensor NCS-1 is a protein the sequence of which is conserved from yeasts to humans.

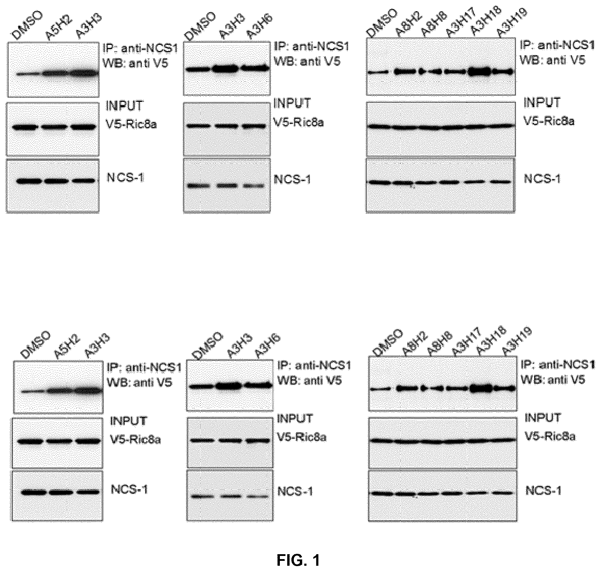

To identify compounds useful as modulators of the protein complex formed by NCS-1 and Ric8 (specifically as compounds useful for promoting NCS-1/Ric8a interaction), the experiment shown in Figure 1 was carried out. Figure 1, specifically the top panel, shows the result of an immunoprecipitation (IP) assay in HEK293 cells transfected with human Ric8a (with V5 tag) and NCS1 in the presence of the compounds identified therein or the vehicle (DMSO). The DMSO lane represents baseline levels of NCS1/Ric8a interaction. IP was performed with anti-NCS1 antibodies, followed by the immunodetection of Ric8a in Western blot with the anti-V5 antibody. The bottom panel shows the input load (1/10 of the lysates before immunoprecipitation). Said IP assay showed that compounds A5H2, A3H3, A3H6, A8H2, A8H8, A3H17, A3H18, and A3H19 promoted NCS-1/Ric8a interaction.

Additional assays were performed with the following list of compounds (see Table 1):

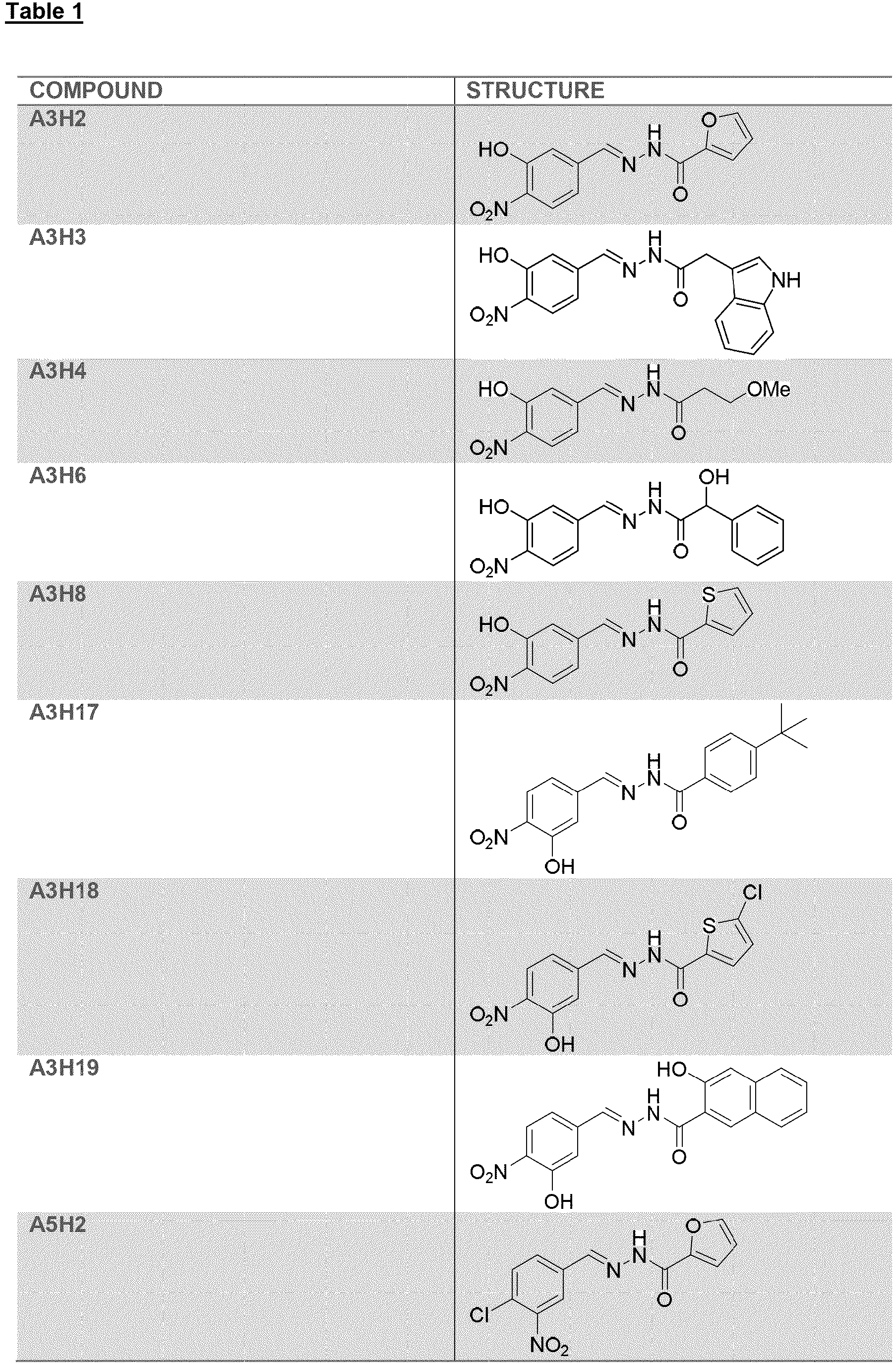

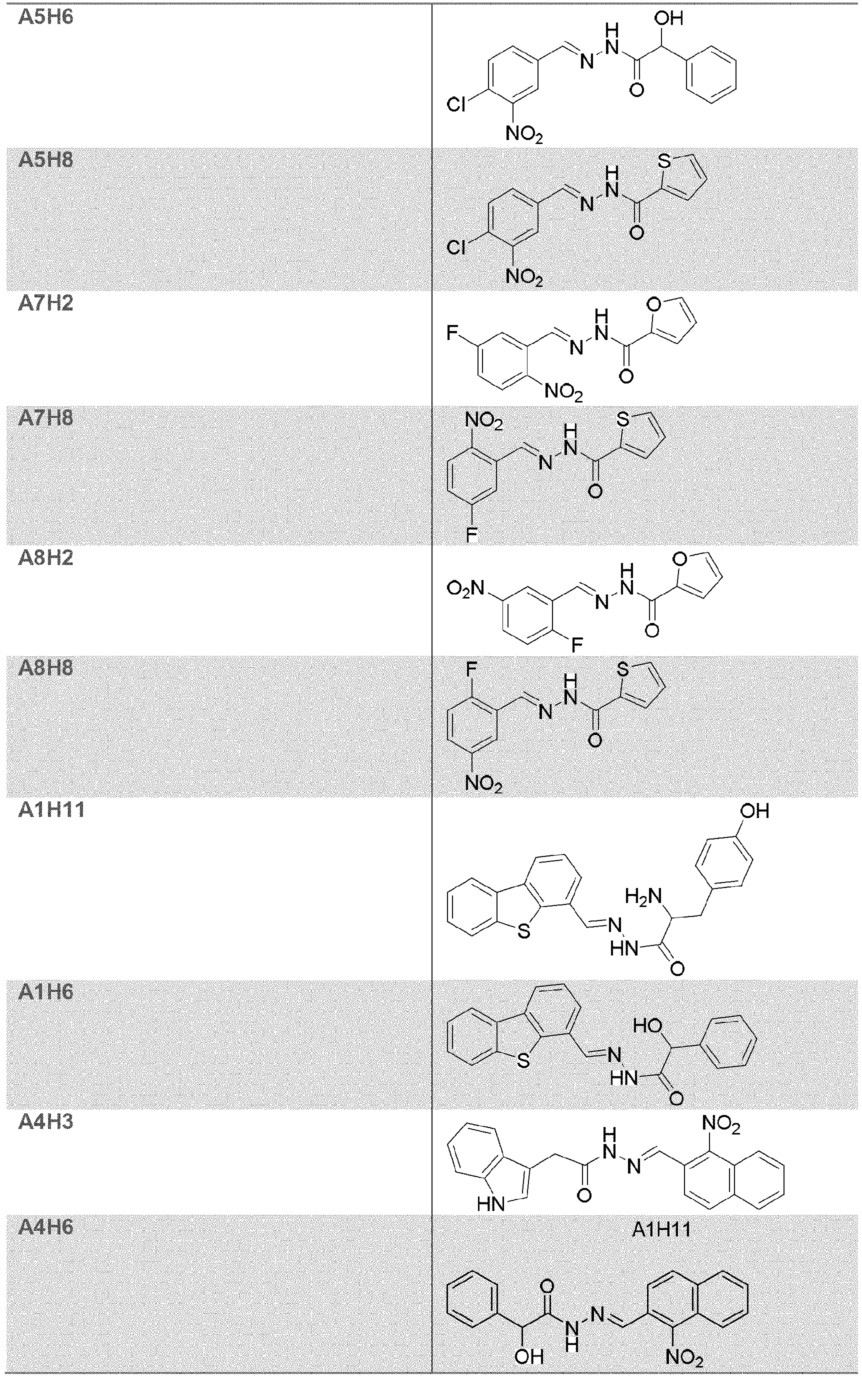

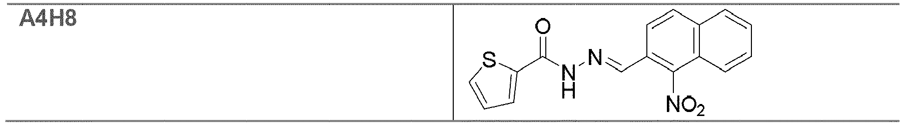

Specifically and in summary, nuclear magnetic resonance (NMR) studies such as STD-NMR (saturation-transfer difference) and tr-NOESY (NOE effect spectroscopy) were conducted from the viewpoint of the ligand. Based on these assays it was determined that A4 type compounds (such as A4H3, A4H6, or A4H8) had to be ruled out due to their low solubility in the assays. Nevertheless, additional fluorescence studies were performed with these compounds without yielding any positive results. Moreover, in these studies it was determined that the derivatives of A1 type compounds were highly insoluble and did not exhibit affinity in the fluorescence technique.

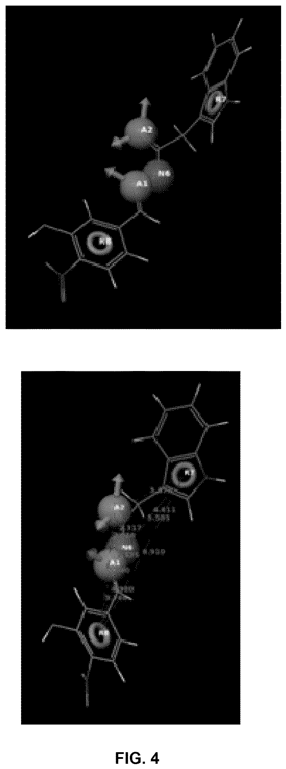

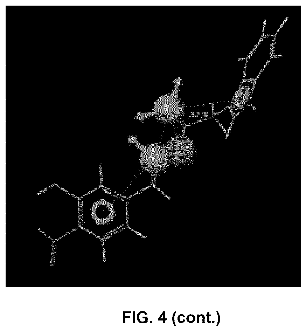

For the rest of the compounds and based on the results discussed above and the fluorescence spectroscopy studies, the structural bases of NCS-1/Ric8a interaction were determined and the pharmacophore group identified. In this sense and as shown in Figure 4, chemical groups A1, A2, and N6 refer to those heteroatoms that form the central part of acylhydrazone commonly found in all the assayed compounds and have proven useful as modulators (activators or promoters) of the protein complex formed by NCS-1 and Ric8. In other words, they refer to the carbonyl (A2) bound to the nitrogen of the amide (N6), which is in turn bound to another imine nitrogen (A1). The terms R7 and R8 refer to aromatic rings acting as substituents. Both the carbonyl-bound group R7 and the imine nitrogen-bound R8 are aromatic. Figure 4 shows compound A3H3 as an example. The information indicated in this paragraph is summarized below:

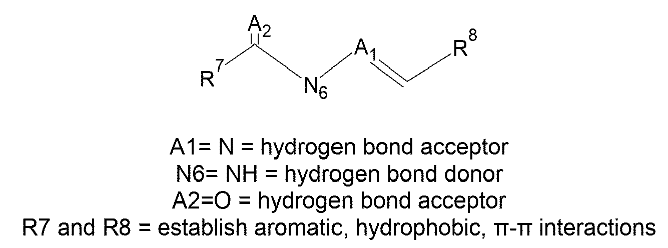

Therefore, the pharmacophore group includes the presence of two aromatic rings (R7 and R8) preferably at the distances and angles indicated in Table 2 shown below, with a spacer consisting of two hydrogen bond acceptors (groups A1, A2) and a group with a negative charge density (group N6). Rigid spacer (CO-NH-N=).

For the compound to act promoting NCS-1/Ric8a interaction, the first aromatic core (R8) must preferably interact with the region of the hNCS1 protein defined by amino acids V68, Y52, F72, F48, W103, and T92, with which π-π type hydrophobic interactions are established. Furthermore, group R8 must preferably be located perpendicular to the molecular surface of the protein so as to expose the polar groups to the solvent through the ring substituents.

For the compound to act promoting NCS-1/Ric8a interaction, the second aromatic core (R7) must preferably interact with the N-terminus part of the hydrophobic cleft of the NCS1 protein, on one side with W30, F85, and L89, and on the other side with 151 and F55, through which π-π type hydrophobic interactions must be established. It should be noted that π-π interactions occur between two aromatic groups, the pi electron clouds of which "recognize" one another. These interactions are stronger and more stabilizing than a hydrophobic interaction. Since groups R7 and R8 contain, or may contain, an aromatic residue, it interacts with other aromatic groups by means of π-π interactions. Those hydrophobic-non-aromatic residues of R7 and/or R8 would interact through what is referred to as hydrophobic interactions. In other words, π-π interactions in the context of R7 or R8 must occur with a specific geometry between rings. When this geometry does not occur, the interactions are generally said to be hydrophobic.

**Table 2. Bond distances and angles**

| **Site 1** | **Site 2** | **Distance (Å)** | | **Site 1** | **Site 2** | **Site 3** | **Angle (°)** |
|---|---|---|---|---|---|---|---|
| | | | | A2 | A1 | N6 | 61.8 |
| A1 | A2 | 2.626 | | A2 | A1 | R7 | 38.1 |
| A1 | N6 | 1.325 | | A2 | A1 | R8 | 141.1 |
| A1 | R7 | 5.585 | | N6 | A1 | R7 | 37.8 |
| A1 | R8 | 3.766 | | N6 | A1 | R8 | 152.5 |
| A2 | N6 | 2.317 | | R7 | A1 | R8 | 143.9 |
| A2 | R7 | 3.874 | | A1 | A2 | N6 | 30.3 |
| A2 | R8 | 6.04 | | A1 | A2 | R7 | 117.2 |
| N6 | R7 | 4.611 | | A1 | A2 | R8 | 23 |
| N6 | R8 | 4.98 | | N6 | A2 | R7 | 92.8 |
| R7 | R8 | 8.91 | | N6 | A2 | R8 | 52.5 |
| | | | | R7 | A2 | R8 | 126.6 |
| | | | | A1 | N6 | A2 | 87.9 |
| | | | | A1 | N6 | R7 | 132 |
| | | | | A1 | N6 | R8 | 20.4 |
| | | | | A2 | N6 | R7 | 57 |
| | | | | A2 | N6 | R8 | 105.9 |
| | | | | R7 | N6 | R8 | 136.5 |
| | | | | A1 | R7 | A2 | 24.7 |
| | | | | A1 | R7 | N6 | 10.1 |
| | | | | A1 | R7 | R8 | 14.4 |
| | | | | A2 | R7 | N6 | 30.1 |
| | | | | N6 | R8 | R7 | 20.8 |

It should be noted that it is possible to determine if a compound interacts with the region of the NCS1 protein defined by amino acids V68, Y52, F72, F48, W103, and T92, and/or with the region bounded by amino acids W30, F85, and L89 on one side, and I51 and F55 on the other side of the NCS1 protein, by knowing the three-dimensional (crystalline) structure of the protein and specifically the two regions specified above. In this sense, the crystalline structure of the Drosophila protein is contained in PDB ID code 4BY4, molecule B (https://www.rcsb.org/structure/4by4). As illustrated below, this structure is valid for determining if a compound interacts with the region of the human NCS1 protein defined by amino acids V68, Y52, F72, F48, W103, and T92, and/or with the region bounded by amino acids W30, F85, and L89 on one side, and I51 and F55 on the other side of the NCS1 protein, given that although there is a 70.6% match in the alignment of the two sequences (human sequence and Drosophila sequence), in the case of the binding pockets of the protein there is a 100% match. To facilitate understanding of the foregoing, the amino acids intervening in said pockets have been marked below, where the amino acids for Drosophila are in bold and the amino acids for human are underlined. The participating amino acids are: Y52, V68, F72, T92, W103, W30, F85, L89, F55, F48, and 151.

sp|P62166|NCS1_HUMAN
MGKSNSKLKPEVVEELTRKTYFTEKEVQQWYKGFIKDCPSGQLDAAGFQKIYKQFFPFGD 60
tr|Q9VWX8|Q9VWX8_DROME
MGKKNSKLKQDTIDRLTTDTYFTEKEIRQWHKGFLKDCPNGLLTEQGFIKIYKQFFPDGD 60
***.***** :.::.**
.*******::**:***:****.* *   ** ******** **

sp|P62166|NCS1_HUMAN
PTKFATFVFNVFDENKDGRIEFSEFIQALSVTSRGTLDEKLRWAFKLYDLDNDGYITRNE 120
tr|Q9VWX8|Q9VWX8_DROME
PSKFASLVFRVFDENNDGAIEFEEFIRALSITSRGNLDEKLHWAFRLYDVDNDGYITREE 120
*:***::**.*****:**
***.***:***:****.*****:***:***:********:*

sp|P62166|NCS1 HUMAN
MLDIVDAIYQMVGNTVELPEEENTPEKRVDRIFAMMDKNADGKLTLQEFQEGSKADPSIV 180
tr|Q9VWX8|Q9VWX8 DROME
MYNIVDAIYQMVGQQPQ-TEDENTPQKRVDKIFDQMDKNHDDRLTLEEFREGSKADPRIV 179
* :**********: : *:****:****:** ****
*.:***:**:******* **

sp|P62166|NCS1 HUMAN          QALSLYDGLV190
tr|Q9VWX8|Q9VWX8 DROME        QALSLGGD--187
                              *****  ..

1: sp|P62166|NCS1_HUMAN
2: tr|Q9VWX8|Q9VWX8_DROME
Identity: 69.5%
Similarity 81.1%

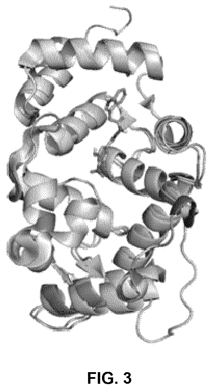

All this data that is shown referring to sequence identity and similarity between hNCS-1 and *Drosophila melanogaster* frequenin-2 (dNCS-1) were calculated by means of pair alignment using the EMBOSS Needle tool (https://www.ebi.ac.uk/Tools/emboss/). Figure 3 shows this data together with the superposition of the crystallographic structures of hNCS-1 (yellow, PDB yet to be published) bound to 3b (green) and dNCS-1 (blue, PDB 5AAN). The residues in which sequence differences are found are shown in pink.

Therefore, compounds useful for promoting NCS-1/Ric8a interaction are characterized in that they comprise a pharmacophore group comprising the following chemical structure: 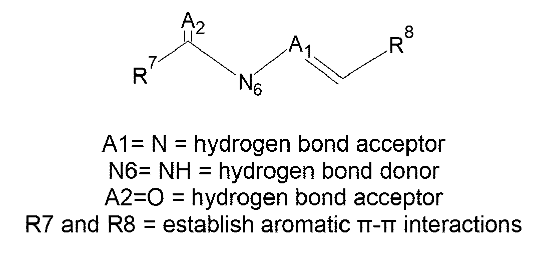 wherein R7 and R8 are groups including the presence of two aromatic rings (R7 and R8), and wherein preferably A1 is at a distance from A2 of 2.63 ± 0.25 A, wherein preferably A1 is at a distance from N6 of 1.33 ± 0.15 Å, wherein preferably A2 is at a distance from N6 of 2.32 ± 0.25 Å, wherein preferably A1 is at a distance from R7 of 5.58 ± 0.55 Å, wherein preferably A1 is at a distance from R8 of 3.77 ± 0.35 Å, wherein preferably A2 is at a distance from R7 of 3.87 ± 0.35 Å, wherein preferably A2 is at a distance from R8 of 6.04 ± 0.50 Å, wherein preferably N6 is at a distance from R7 of 4.61 ± 0.45 Å, and wherein preferably N6 is at a distance from R8 of 4.98 ± 0.35 Å.

It should be noted that in the context of the present invention, the decimal point is used, i.e., a period is used to separate decimals and a comma to separate thousands.

In the context of the present invention, the distance represents distances between the centers of the respective atoms. It should be noted that the distance with groups R7 or R8 refers to the distance between the center of the heteroatom of group A1, A2, or N6 and the center of the first atom of R7 or R8 bound through a covalent bond with the N of group A1 or with the carbon of group A2. One skilled in the art will know the ways to measure said distances, which would include, for example, by means of the Maestro program (Schrödinger).

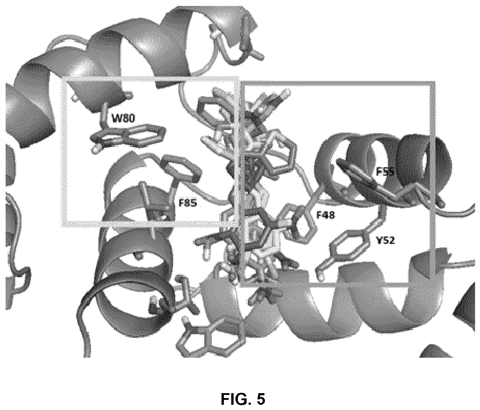

The pharmacophore group is therefore clearly defined for those compounds useful for promoting NCS-1/Ric8a interaction; nevertheless, the chemical structure of groups R7 and R8 of the pharmacophore group would have to be specified. To that end, compounds A3H3, A5H6, and A7H2 were modeled (given that all three exhibited modulatory activity) in Drosophila Frq2 based on the x-ray crystallographic structure of said protein (see Figure 5 Drosophila Frq2 (PDB 4by4)). With this modeling, it could be observed that group R8 requires the presence of an aromatic group, preferably a group derived from a nitrobenzaldehyde (with ortho-, meta-, or para-substitution of the nitro group) and with the possibility of weak donor groups or donors capable of establishing hydrogen bonds. It is important to establish that the presence in R8 of an aromatic group with two or three fused rings causes the compound to lose affinity, and therefore group R8 must be limited to a single aromatic ring. With respect to group R7, both simple and branched linear alkyl chains are ruled out given that the activity of the compound is reduced. In contrast, when group R7 is formed by a heteroatom with 2 fused rings, i.e., heterocyclic compounds such as the indole group, or by a single heterocyclic ring (such as a furan or thiophene) with the possibility of bulky substituents, the activity is increased significantly as observed by means of the fluorescence technique.

Therefore, group R8 must be an aromatic ring, such as a benzyl, preferably ortho-, meta-, or para-substituted with a nitro group and ortho-, meta-, or para-substituted with donor groups capable of establishing hydrogen bonds such as F, Cl, Br, CF₃, or a hydroxyl group.

Furthermore, group R8 must preferably be capable of interacting with the region of the NCS1 protein defined by amino acids V68, Y52, F72, F48, W103, and T92.

Moreover, group R7 must be a heterocycle with 2 fused rings, i.e., heterocyclic compounds such as the indole group, or a single heterocyclic ring (such as a furan or thiophene), with the possibility of bulky substituents, or -[CH₂]ₙ-R₆, wherein n has any of the following values: 0, 1, or 2; and wherein R6 is a heterocycle with 2 fused rings, i.e., heterocyclic compounds such as the indole group, or a single heterocyclic ring (such as a furan or thiophene). Furthermore, group R7 must preferably interact with the region bounded by amino acids W30, F85, and L89, and on the other side with I51 and F55, through which π-π type hydrophobic interactions must be established.

Finally, it must be indicated that as can be seen in Figure 5 the h10 α helix of the NCS-1 protein plays a fundamental role in NCS-1/Ric8a interaction, given that it is a competitive inhibitor of said NCS-1/Ric8a interaction and works by way of a gatekeeper, regulating protein accessibility to the hydrophobic cleft (Romero-Pozuelo *et al.,* 2014). This dynamic is the key to regulation. The formation of the complex increases the number of synapses. Based on this mechanism, the stabilizers described in the preceding paragraphs keep the helix out, selecting an NCS-1 conformational state that favors interaction with Ric8a.

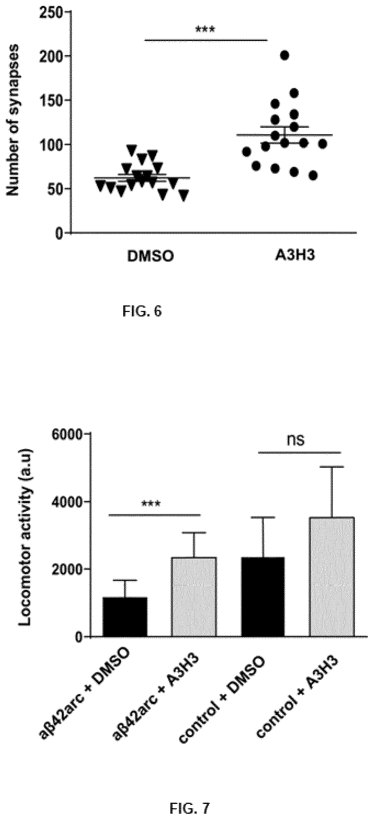

In order to validate the results discussed in the preceding paragraphs, Figure 6 shows an example of the effect of one of the candidate compounds on promoting the number of synapses in a *Drosophila melanogaster* model for Alzheimer's. It has been described above that the expression of the human aβ42arc peptide (Arctic mutation) in motor neurons leads to a reduction in the number of synapses with respect to normal neuromuscular junctions in individuals of the same age, therefore, flies overexpressing human aβ42arc (D42Gal4> aβ42arc) were fed with 100 µM compound A3H3 or with the same amount of solvent, DMSO, throughout the entire life cycle. Adult abdominal motor neurons of 20 days were analyzed and the number of synapses (nc82-positive points) was determined in different flies using confocal images.

The data confirmed that the pathological reduction of the synapse count was greatly suppressed in those flies fed with compound A3H3.

Figure 7 shows the functional impact of synapse recovery in a *Drosophila melanogaster* model. As mentioned, overexpression of the human aβ42arc peptide in the motor neurons of *Drosophila melanogaster* causes synapse loss and this correlates with a severe locomotor dysfunction. Flies overexpressing aβ42arc (D42Gal4> aβ42arc) in their motor neurons or overexpressing the control (D42Gal4> LacZ) were fed with compound A3H3 at a concentration of 100 µM or with DMSO using the same concentration. Fly locomotor activity was recorded in Drosophila activity monitors (DAM2, Trikinetics) and the total number of beam breaks per hour was analyzed for two consecutive days. It must be pointed out that locomotor impairment due to overexpression of aβ42arc thoroughly improved by feeding A3H3. Furthermore, the statistical analysis of the data did not reveal any significant difference in the locomotor activity of the control flies fed with A3H3 in comparison with those fed with DMSO.

Therefore, in a first aspect the present invention relates to a compound suitable for promoting NCS-1 and Ric8a interaction, wherein said interaction can be verified by means of co-immunoprecipitation assays, and said compound comprises the structure of formula (I): 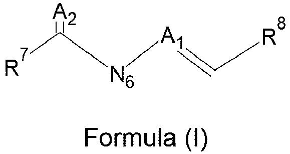 wherein A1 is nitrogen, wherein N6 is NH, and wherein A2 is oxygen;
wherein preferably, but in a non-limiting manner, in formula I, A1 is at a distance from A2 of 2.63 ± 0.25 Å, wherein preferably A1 is at a distance from N6 of 1.33 ± 0.15 Å, wherein preferably A2 is at a distance from N6 of 2.32 ± 0.25 Å, wherein preferably A1 is at a distance from R7 of 5.58 ± 0.55 Å, wherein preferably A1 is at a distance from R8 of 3.77 ± 0.35 Å, wherein preferably A2 is at a distance from R7 of 3.87 ± 0.35 Å, wherein preferably A2 is at a distance from R8 of 6.04 ± 0.50 Å, wherein preferably N6 is at a distance from R7 of 4.61 ± 0.45 Å, and wherein preferably N6 is at a distance from R8 of 4.98 ± 0.35 Å;
wherein R7 must preferably interact with the N-terminus part of the hydrophobic cleft of the NCS1 protein (PDB ID code 4BY4, molecule B (https://www.rcsb.org/structure/4by4)), on one side with W30, F85, and L89, and on the other side with 151 and F55, through which π-π type hydrophobic interactions must be established, and comprises the following chemical structure selected from the list consisting of:
- a heteroaryl with a single ring, preferably a thiophene or a furan;
- a heteroaryl with two fused rings, preferably an indole group; or
- a -[CH2]ₙ-R6 group, wherein n has any of the following values: 0, 1, or 2; and wherein R6 is a heterocycle with 2 fused rings or a single heterocyclic ring; and
wherein R8 preferably interacts with the region of the NCS1 protein (PDB ID code 4BY4, molecule B (https://www.rcsb.org/structure/4by4)) defined by amino acids V68, Y52, F72, F48, W103, and T92, with which π-π type hydrophobic interactions are established, and comprises the structure of formula II: 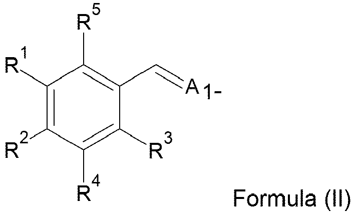 wherein at least one of the R¹ to R⁵ radicals of the structure of formula (II) is a nitro group and another one of the radicals is selected from the list consisting of: H, F, Cl, Br, CF₃, or a hydroxyl group; wherein the compound of formula (II) binds to the rest of the compound of formula (I) through the methine or methylidene group which in turn binds to group A1;
or any of the isomers or salts thereof, for use in promoting interaction between NCS-1 and Ric8a proteins involved in the process of regulating the number of synapses in those diseases presenting with a reduction in the number of neuron synapses, preferably wherein said diseases are selected from the list consisting of Alzheimer's disease, Parkinson's disease, Huntington's disease, and other neurodegenerative diseases, as well as Angelman syndrome, fragile X chromosome syndrome, Rett syndrome, syndromes of the autism spectrum, epilepsy, schizophrenia, bipolar disorder, amyotrophic lateral sclerosis, Friedreich ataxia, progressive supranuclear palsy, frontotemporal dementia, Lewy body dementia, Creutzfeldt-Jakob disease. More preferably, the disease is Alzheimer's disease.

In a preferred embodiment, R¹ is selected from OH, NO₂, F, Cl, or Br and wherein R² to R³ are selected from the list consisting of H, NO₂, F, Cl, or Br.

In a preferred embodiment, R¹ is OH.

In another preferred embodiment, R² is NO₂.

In another more preferred embodiment, when R¹ is OH, R₂ is NO₂.

In another preferred embodiment, R³ is H.

In another even more preferred embodiment, when R¹ is OH and R² is NO₂, R₃, R₄, and R₅ are H.

In another preferred embodiment, R¹ is NO₂.

In another preferred embodiment, R² is F, Cl, or Br, preferably R₂ is chlorine.

In another more preferred embodiment, when R¹ is NO₂, R² is F, Cl, or Br, preferably R² is chlorine.

In another even more preferred embodiment, when R¹ is NO₂ and R² is F, Cl, or Br, preferably R² is chlorine, R³ is H.

In another more preferred embodiment, when R¹ is NO₂, R² is H.

In another even more preferred embodiment, when R¹ is NO₂ and R² is H, R³ is F, Cl, or Br, preferably R³ is fluorine.

In another preferred embodiment, R¹ is F, Cl, or Br, preferably R¹ is fluorine.

In another preferred embodiment, R² is H.

In another more preferred embodiment, when R¹ is F, Cl, or Br, preferably fluorine, R² is H.

In another even more preferred embodiment, when R¹ is F, Cl, or Br, preferably R¹ is fluorine, and R² is H, R³ is NO₂.

In another preferred embodiment, R⁷ is a heteroaryl which is selected from thiophenyl or furanyl, wherein said groups can be optionally substituted with halogen.

In another preferred embodiment, R¹ is OH and R² is NO₂ and R³, R⁴, and R⁵ are H.

In another preferred embodiment, R¹ is NO₂, R³ is F, Cl, or Br, preferably fluorine, and R², R⁴, and R⁵ are H.

In another preferred embodiment, R² is NO₂ and optionally R⁴ is OH, the rest of the radicals of R8 are hydrogen.

In another preferred embodiment, R³ is NO₂ and optionally R¹ is F, Cl, or Br, preferably fluorine, the rest of the radicals of R8 are hydrogen.

In another preferred embodiment, R₄ is NO₂ and optionally R² or R³ is F, Cl, or Br, preferably chlorine, the rest of the radicals of R8 are hydrogen.

In another preferred embodiment, R¹ is NO₂ and optionally R⁴ is a halogen, preferably fluorine, the rest of the radicals of R8 are hydrogen.

In another preferred embodiment, R¹ is NO₂ and optionally R² is F, Cl, or Br, preferably chlorine, the rest of the radicals of R8 are hydrogen.

In another preferred embodiment, R¹ is NO₂ and optionally R⁴ is a hydroxyl.

In another preferred embodiment, the compound of formula (I) for use as described above is selected from the following list:
- (*E*/*Z*)-*N'*-(3-hydroxy-4-nitrobenzylidene)-2-(1H-indol-3-yl)acetohydrazide (A3H3)
- (±)-(*E*/*Z*)-2-hydroxy-*N'*-(3-hydroxy-4-nitrobenzylidene)-2-phenylacetohydrazide (A3H6)
- (*E*)-4-(*tert*-butyl)-*N'*-(3-hydroxy-4-nitrobenzylidene)benzohydrazide (A3H17)
- (*Z*)-5-chloro-*N'*-(3-hydroxy-4-nitrobenzylidene)thiophene-2-carbohydrazide (A3H18)
- (*E*/*Z*)-3-hydroxy-*N*'-(3-hydroxy-4-nitrobenzylidene)-2-naphthohydrazide (A3H19)
- (*Z*)-*N*'-(4-chloro-3-nitrobenzylidene)furan-2-carbohydrazide (A5H2)
- (*E*/*Z*)-*N*'-(4-chloro-3-nitrobenzylidene)thiophene-2-carbohydrazide (A5H8)
- (*E*)-*N*'-(5-fluoro-2-nitrobenzylidene)furan-2-carbohydrazide (A7H2)
- (*E*/*Z*)-*N*-(5-fluoro-2-nitrobenzylidene)thiophene-2-carbohydrazide (A7H8)
- (*E*)-*N*'-(2-fluoro-5-nitrobenzylidene)furan-2-carbohydrazide (A8H2)
- (*E*)-(*N*'-(2-fluoro-5-nitrobenzylidene)-2-hydroxy-2-phenylacetohydrazide (A8H8)

A second aspect of the invention relates to a compound which is selected from:
- (*E*/*Z*)-*N*'-(3-hydroxy-4-nitrobenzylidene)-2-(1H-indol-3-yl)acetohydrazide (A3H3)
- (*E*)-4-(*tert*-butyl)-*N*'-(3-hydroxy-4-nitrobenzylidene)benzohydrazide (A3H17)
- (*Z*)-5-chloro-*N'*-(3-hydroxy-4-nitrobenzylidene)thiophene-2-carbohydrazide (A3H18)
- (*E*/*Z*)-3-hydroxy-*N*'-(3-hydroxy-4-nitrobenzylidene)-2-naphthohydrazide (A3H19)
- (*E*)-*N*'-(5-fluoro-2-nitrobenzylidene)furan-2-carbohydrazide (A7H2)
- (*E*/*Z*)-*N*-(5-fluoro-2-nitrobenzylidene)thiophene-2-carbohydrazide (A7H8)
- (*E*)-*N*'-(2-fluoro-5-nitrobenzylidene)furan-2-carbohydrazide (A8H2)
- (*E*)-(*N*'-(2-fluoro-5-nitrobenzylidene)-2-hydroxy-2-phenylacetohydrazide (A8H8)

A third aspect of the invention relates to a compound of the second aspect of the invention for use as a medicinal product.

A fourth aspect of the invention relates to a pharmaceutical composition comprising at least one compound of the second aspect of the invention and at least one pharmaceutically acceptable vehicle.

The term "aryl" refers herein to a radical phenyl, naphthyl, indenyl, phenanthryl, or anthracyl, preferably phenyl and naphthyl. The aryl radical can be optionally substituted with one or more substituents such as alkyl, haloalkyl, aminoalkyl, dialkylamino, hydroxyl, alkoxyl, phenyl, mercapto, halogen, nitro, cyano, and alkoxycarbonyl.

The term "heteroaryl" refers to an aryl as defined above containing at least one atom other than carbon, such as S, N, or O, being part of the aromatic ring. Examples of heteroaryl radicals include thiophenyl, furanyl, pyrrolyl, pyrazolyl, imidazolyl, thiazolyl, isothiazolyl, oxazolyl, isoxazolyl, triazolyl, thiadiazolyl, oxadiazolyl, pyridinyl, pyrazinyl, pyridazinyl, pyrimidinyl, triazinyl, thienofuranyl, thienopyrrolyl, pyrrolopyrazolyl, benzothiophenyl, benzofuranyl, indolyl, isoindolyl, benzimidazolyl, benzothiazolyl, imidazopyridinyl, pyrazolopyridinyl, isoquinolinyl, quinolinyl, quinolizinyl, naphthyridinyl, quinazolinyl, quinoxalinyl. The heteroaryl groups can be optionally substituted in any of their positions with one or more substituents or with two substituents, forming a cycle condensed to the heteroaryl and said substituents are independently selected from CF₃, C₁-C₆ alkyl, S-C₁-C₆ alkyl, halogen, CN, O-C₁-C₆ alkyl, NO₂, COO-C₁-C₆ alkyl, NHCO-C₁-C₆ alkyl, NH₂, and NH-C₁-C₆ alkyl, and more preferably CF₃, C₁-C₆ alkyl, halogen, CN, and NO₂.

The compounds of the invention, in therapeutic use or being part of a pharmaceutical composition, can be in crystalline form as free compounds or solvates and it is intended that both forms are within the scope of the present invention. In this sense, the term "solvate" as it is used herein, includes both pharmaceutically acceptable solvates which can be used in the production of a medicinal product, and non-pharmaceutically acceptable solvates which can be useful in the preparation of pharmaceutically acceptable salts or solvates. The nature of the solvate pharmaceutically acceptable solvate is not critical provided that it is pharmaceutically acceptable. In a particular embodiment, the solvate is a hydrate. The solvates can be obtained by conventional solvation methods well known by those skilled in the art.

The compounds of the invention for therapeutic use or being part of a pharmaceutical composition are prepared in the form of a solid or an aqueous suspension, in a pharmaceutically acceptable diluent. These preparations can be administered through any suitable route of administration, for which said preparation will be formulated in the dosage form suited to the chosen route of administration. In a particular embodiment, the compound of formula (I) provided by this invention is administered orally, topically, rectally, or parenterally (including subcutaneously, intraperitoneally, intradermally, intramuscularly, intravenously, etc.). The preparation of the different medicinal product administration dosage and the excipients required for obtaining same can be found in different manuals handled by those skilled in the art.

The compounds described in the present invention, their pharmaceutically acceptable salts, solvates, as well as the pharmaceutical compositions containing same can be used together with other additional drugs to provide a combination therapy. Said additional drugs can be part of the same pharmaceutical composition, or alternatively can be provided in the form of a separate composition for simultaneous or non-simultaneous administration with the pharmaceutical composition comprising a compound of the invention, or a pharmaceutically acceptable salt, stereoisomer, or solvate thereof.

Unless otherwise indicated, the compounds of the invention also include compounds that only differ in the presence of one or more isotopically enriched atoms. For example, compounds having said structure, with the exception of the substitution of a hydrogen with a deuterium or tritium, or the substitution of a carbon with a carbon enriched with ¹³C or ¹⁴C or a nitrogen enriched with ¹⁵N, are within the scope of this invention.

Throughout the description and claims, the word "comprises" and variants thereof do not seek to exclude other technical features, additions, components, or steps. For those skilled in the art, other objects, advantages, and features of the invention will be inferred in part from the description and in part from the practice of the invention. The following examples and drawings are provided by way of illustration and do not seek to limit the present invention.

### BRIEF DESCRIPTION OF THE DRAWINGS

**Figure 1****.** This figure shows the results of immunoprecipitation assays in HEK293 cells transfected with human V5-Ric8a and NCS-1 and in the presence or absence of the compounds. The DMSO lane represents a positive control of the interaction.
**Figure 2****.** This figure shows the depiction of the fluorescence emission of the dNCS-1-ligand complex in the presence of calcium, in increasing concentrations of ligand. The curves were fitted considering a stoichiometry of 1:1. For correct curve comparison, intensities were normalized and depicted as (I₀ - I)/I₀. X. The value of r² is 0.998.
**Figure 3****.** This figure shows the superposition of the crystallographic structures of hNCS-1 (yellow, PDB) bound to 3b (green) and dNCS-1 (blue, PDB 5AAN). The residues in which sequence differences are found are shown in pink.
**Figure 4****.** These three figures show the pharmacophore group taking the compound A3H3 as an example. Specifically, groups A1, A2, and N6 (N) refer to the heteroatoms forming the central part of acylhydrazone commonly found in all the assayed compounds, in other words, the carbonyl (A1) bound to the N of amide (N6), which is in turn bound to another imine N (A2). The terms R7 (R) and R8 (R') refer to the aromatic rings which act as substituents. Both the carbonyl-bound R and the imine N-bound R are aromatic
**Figure 5****.** This figure shows the x-ray crystallographic structure of Drosophila Frq2 (PDB 4by4) to which compounds A3H3, A5H6, and A7H2 oriented towards pocket 1 and pocket 2 have been superposed.
**Figure 6****.** This figure shows an example of the effect of one of the candidate compounds on promoting the number of synapses in a *Drosophila melanogaster* model for Alzheimer's disease.
**Figure 7****.** This figure shows the functional impact of synapse recovery in a *Drosophila melanogaster* model for Alzheimer's disease.

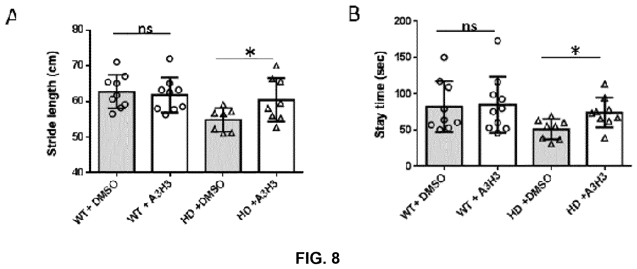

**Figure 8****.** Evaluation of the efficacy of compound A3H3 in a model for Huntington's disease. Wild-type mice WT and transgenic mice (HD) expressing exon 1 of human huntingtin (strain R6/1) were treated orally with compound A3H3 at a dose of 25 mg/kg. The treatment started at 3 months of age and lasted for 6 weeks. As a measurement of motor coordination, stride length (A) and the stay time on Rotarod (B) were analyzed. The mice did not show differences in any of these two parameters at the start of the treatment, however after 6 weeks (A and B), HD mice treated with the vehicle DMSO show significant differences with respect to the rest of groups, but the same cannot be said for HD mice treated with compound A3H3.

### EXAMPLES

The invention will be illustrated below by means of assays performed by the inventors which clearly demonstrate the effectiveness of the product of the invention.

### Example 1. Synthesis of the compounds of the invention

### Preparing and obtaining (E)-N'-(3-hydroxy-4-nitrobenzylidene)furan-2-carbahydrazide (A3H2)

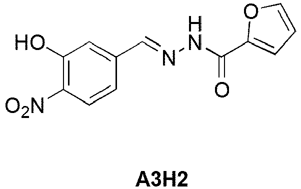

3-hydroxy-4-nitrobenzaldehyde (319 mg, 1.91 mmol), 2-furoic acid hydrazide (200 mg, 1.59 mmol), MeOH (60 ml). The product is filtered and washed with MeOH at room temperature to obtain the compound as a yellow solid (0.30 g, 70%) (E > 99). **mp:** 256 - 257°C. **¹H-NMR** (300 MHz, DMSO-*d₆*) δ 12.07 (s, 1H), 11.18 (s, 1H), 8.40 (s, 1H), 7.98 (m, 2H), 7.49 (d, *J* = 1.7 Hz, 1H), 7.40 - 7.26 (m, 2H), 6.72 (d, 1H, *J* = 1.7 Hz). **¹³C-NMR** (75 MHz, DMSO-*d₆*): δ 164.5, 152.3, 146.7, 145.2, 140.5, 140.0, 137.2, 125.9, 118.3, 118.5, 116.8, 112.6. **Anal. Calc.** for C₁₂H₉N₃O₅: C, 52.37%; H, 3.30%; N, 15.27%. **Found:** C, 52.43%; H, 3.30%; N, 15.11%. **HPLC-MS:** t_{R}: 7.02 min (ACE-Excel 3 C18-PFP column, gradient 15-85% H₂O:CH₃CN 0.1% formic acid), [M+H]⁺ = 276 m/z.

### Preparing and obtaining (E/Z)-N'-(3-hydroxy-4-nitrobenzylidene)-2-(1H-indol-3-yl)acetohydrazide (A3H3)

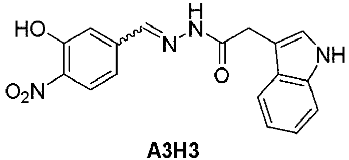

3-hydroxy-4-nitrobenzaldehyde (330 mg, 1.97 mmol), indole-3-acetichydrazide (300 mg, 1.58 mmol), MeOH (60 ml). The product is filtered and washed with MeOH for obtaining same as an orange solid (0.30 g, 70%) (E:Z = 40:60). mp: 216 - 217°C. **¹H-NMR** (300 MHz, DMSO-d₆) δ 11.79 (s, 1H, E), 11.54 (s, 1H, Z), 11.19 (s, 1H), 7.88 (s, 1H), 7.70-7.65 (m, 1H), 7.39-7.36 (m, 1H), 7.27-7.25 (m, 1H), 7.15(s, 1H), 7.02-6.98 (m, 1H), 6.92-6.88 (m, 1H), 6.78 (s, 1H), 6.72-6.68 (m, 1H), 5.89 (s, 1H), 3.68 (s, 2H). **¹³C-NMR** (75 MHz, DMSO-*d₆*): δ 172.1, 150.8, 148.1, 142.8, 137.4, 136.4, 130.5, 125.9, 123.9, 122.6, 121.0, 120.2, 119.2, 116.7, 111.9, 110.6, 42.5. Anal. Calc. for C₁₇H₁₄N₄O₄: C, 60.35%; H, 4.17%; N, 16.56%. Found: C, 60.13%; H, 4.20%; N, 16.56%. HPLC-MS: t_{R}: 8.45 min (ACE-Excel 3 C18-PFP column, gradient 15-85% H₂O:CH₃CN 0.1% formic acid), [M+H]⁺ = 339 m/z.

### Preparing and obtaining (E)-N'-(3-hydroxy-4-nitrobenzylidene)-3-methoxypropanehydrazide (A3H4)

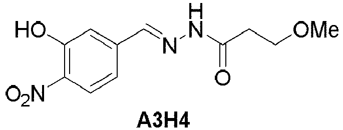

3-hydroxy-4-nitrobenzaldehyde (339 mg, 2.0 mmol), 3-methoxypropionic acid hydrazide (180 µL, 1.69 mmol), MeOH (60 ml). It was purified by means of a Biotage system automated column chromatography using DCM/MeOH (0- 6%) as eluents, obtaining a yellow solid (437 mg, 97%) (E > 99). **mp:** 163 - 164°C. **¹H-NMR** (300 MHz, DMSO-*d₆*): δ 11.61 (s, 1H), 11.13 (s, 2H), 8.13 (s, 1H), 7.95-7.92 (m, 3H), 3.66-3.58 (m, 2H), 3.24 (s, 3H), 2.47 (t, 2H, *J* = 6.8 *Hz*). **¹³C-NMR** (75 MHz, DMSO-*d₆*): δ 172.1, 151.8, 143.1, 140.2, 139.8, 136.6, 125.3, 117.4, 67.4, 57.4, 34.4. **Anal. Calc.** for C₁₁H₁₃N₃O₅: C, 49.44%; H, 4.90%; N, 15.72%. **Found:** C, 49.51%; H, 4.82%; N, 15.61%. **HPLC-MS:** t_{R}: 6.47 min (ACE-Excel 3 C18-PFP column, gradient 15-85% H₂O:CH₃CN 0.1% formic acid), [M+H]⁺ = 268 m/z.

### Preparing and obtaining (E)-4-(tert-butyl)-N'-(3-hydroxy-4-nitrobenzylidene)benzohydrazide (A3H17)

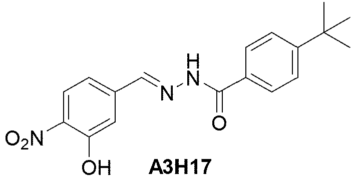

3-hydroxy-4-nitrobenzaldehyde (267 mg, 1.6 mmol), 4-*tert*-butylbenzoic acid hydrazide (255.5 mg, 1.33 mmol), EtOH (60 ml). It is washed and filtered with EtOH to obtain a yellow solid (430 mg, 95%) (E > 99). **mp:** 220-221°C. **¹H-NMR** (300 MHz, DMSO-*d₆*): δ 12.01 (s, 2H), 11.19 (s, 2H), 8.42 (s, 2H), 7.97 (d, *J* = 8.6 Hz, 2H), 7.87 (d, *J* = 8.2 Hz, 5H), 7.61 - 7.49 (m, 7H), 7.31 (d, *J* = 8.6 Hz, 2H), 3.34 (s, 1H), 3.18 (s, 6H), 1.32 (s, 9H). **¹³C-NMR** (75 MHz, DMSO-*d₆*): δ 155.4 (C-1), 152.8 (C-1), 145.5 (C-1), 141.4 (C-1), 137.6 (C-1), 130.8 (C-1), 128.1 (C-1), 126.4 (C-1), 125.7 (C-1), 118.5 (C-1), 116.8 (C-1), 49.1 (C-1), 39.9 (C-1), 35.2 (C-1), 31.4 (C-1).**Anal. Calc.** for C₁₈H₁₉N₃O₄: C, 63.33%; H, 5.61%; N, 12.31%; **Found:** C, 63.10%; H, 6.08%; N, 12.25%. **HPLC-MS:** t_{R}: 9.72 min (ACE-Excel 3 C18-PFP column, gradient 15-85% H₂O:CH₃CN 0.1% formic acid), [M+H]⁺ = 342 m/z.

### Preparing and obtaining (Z)-5-chloro-N'-(3-hydroxy-4-nitrobenzylidene)thiophene-2-carbohydrazide (A3H18)

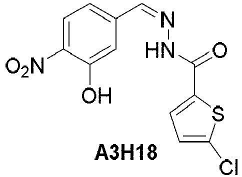

3-hydroxy-4-nitrobenzaldehyde (279 mg, 1.71 mmol), 5-chlorothiophene-2-carboxylic acid hydrazide (249.7 mg, 1.41 mmol), EtOH (60 ml). It is washed and filtered with EtOH to obtain a yellow solid (404 mg, 90%) (Z > 99) **mp:** 255-256°C. **¹H-NMR** (300 MHz, DMSO-*d₆*): δ 12.17 (s, 1H, H-2), 11.28 (s, 1H, H-8), 8.12 - 7.94 (m, 2H, H-10, 14), 7.94 - 7.79 (m, 1H, H-11), 7.51 (m, 1H, H-4), 7.33-7.31 (m, 2H, H-13, 6). **¹³C-NMR** (75 MHz, DMSO-*d₆*): δ 161.2 (C-1),152.8 (C-7),143.3 (C-15),140.3 (C-9),138.2 (C-12),137.9 (C-11), 135.2 (C-10),130.3 (C-5),127.2 (C-4), 126.6 (C-6), 118.6 (C-13), 117.4 (C-14). **Anal. Calc.** for C₁₂H₈ClN₃O₄S: C, 44.25%; H, 2.48%; N, 12.90%; S, 9.84%. Found: C, 44.16%; H, 2.58%; N, 12.85%; S, 9.87%. **HPLC-MS:** t_{R}: 9.48 min (ACE-Excel 3 C18-PFP column, gradient 15-85% H₂O:CH₃CN 0.1% formic acid), [M+H]⁺ = 326 m/z.

### Preparing and obtaining (E/Z)-3-hydroxy-N'-(3-hydroxy-4-nitrobenzylidene)-2-naphthohydrazide (A3H19)

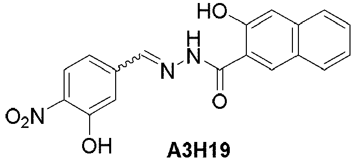

3-hydroxy-4-nitrobenzaldehyde (198 mg, 1.18 mmol), 3-hydroxy-2-naphthoic acid hydrazide (206 mg, 0.98 mmol), EtOH (60 ml). The product is filtered and washed with EtOH to obtain a yellow solid (287 mg, 83%) (E:Z = 88:12). **mp:** 264-265°C. **¹H-NMR** (300 MHz, DMSO-*d₆*): δ 12.13 (s, 1H, H-2, *E*),12.05 (s, 1H, H-2, Z), 11.21 (s, 2H, H-10, 21), 8.44 (m, 2H, H-4, 7), 7.96 (d, *J* = 8.4 Hz, 2H, H-14, 17), 7.78 (d, *J* = 8.3 Hz, 1H, H-6), 7.60 -7.43 (m, 2H, H-15, 16), 7.43 - 7.30 (m, 3H, H-11, 12, 19).**¹³C-NMR** (75 MHz, DMSO-*d₆*): δ 164.2 (C-1), 154.2 (C-20), 152.8 (C-9), 146.4 (C-4), 140.9 (C-8), 137.8 (C-5), 136.3 (C-22), 131.1 (C-18), 129.1 (C-13), 128.7 (C-7), 127.3 (C-12), 126.4 (C-6), 126.3 (C-17), 124.3 (C-14), 121.2 (C-16), 118.6 (C-15), 117.0 (C-11), 111.0 (C-19). **Anal. Calc.** for C₁₈H₁₃N₃O₅: C, 61.54%; H, 3.73%; N, 11.96%. **Found:** C, 61.51%; H, 3.76%; N, 11.97%. **HPLC-MS:** t_{R}: 9.38 min (ACE-Excel 3 C18-PFP column, gradient 15-85% H₂O:CH₃CN 0.1% formic acid), [M+H]⁺ = 353 m/z.

### Preparing and obtaining (E)-N'-(5-fluoro-2-nitrobenzylidene)furan-2-carbohydrazide (A7H2)

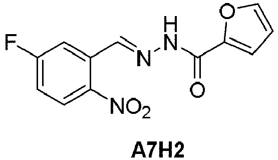

5-fluoro-2-nitrobenzaldehyde (250 mg, 1.5 mmol), 2-furoic acid hydrazide (150 mg, 1.2 mmol), MeOH (50 ml). It is purified by means of a Biotage system automated silica column chromatography, DCM/MeOH (0 - 3%) obtaining a yellow solid (0.330 g, 99%) (E > 99). mp: 194 - 195°C. **¹H-NMR** (300 MHz, DMSO- *d₆*): δ 12.33 (s, 1H, H-2), 8.91 (s, 1H, H-4), 8.23 (dd, *J* = 9.2 Hz, 1H, H-14), 8.00 (d, *J* = 1.8 Hz, 1H, H-7), 7.81 (dd, *J* = 9.6, 2.9 Hz, 1H, H-12), 7.56 (dd, *J* = 9.0, 2.9 Hz, 1H, H-13), 7.40 (d, *J* = 3.4 Hz, 1H, H-10), 6.75 (dd, *J* = 3.4, 1.8 Hz, 1H, H-8). **¹³C-NMR** (75 MHz, DMSO- *d₆*): δ 165.66 (C-1), 162.30 (C-9) 146.31 (C-7), 144.49 (C-4), 132.26 (C-6), 132.19 (C-11), 128.30 (C-14), 128.25 (C-5), 117.58 (C-13), 117.50 (C-10), 113.96 (C-12), 112.16 (C-8). **Anal. Calc.** for C₁₂H₈FN₃O₄: C, 51.99%; H, 2.91%; N, 15.16%. **Found:** C, 51.94%; H, 2.91%; N, 15.00%. **HPLC-MS:** t_{R}: 7.00 min (ACE-Excel 3 C18-PFP column, gradient 15-85% H₂O:CH₃CN 0.1% formic acid), [M+H]⁺ = 278 m/z.

### Preparing and obtaining (E/Z)-N'-(5-fluoro-2-nitrobenzylidene)thiophene-2-carbohydrazide (A7H8)

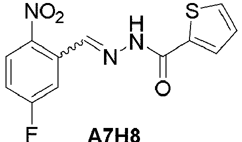

5-fluoro-2-nitrobenzaldehyde (220 mg, 1.3 mmol), 2-thiophenecarboxylic acid hydrazide (160 mg, 1.1 mmol), MeOH (50 ml). The solid obtained by crystallization as an orange solid is filtered and washed (0.270 g, 82%) (E:Z = 26:74). **mp:** 218 - 219°C. **¹H-NMR** (300 MHz, DMSO- *d₆*): δ 12.33 (s, 1H, H-2, E), 12.32 (s, 1H, H-2, Z), 8.89 (s, 1H, H-7), 8.25 (dd, *J* = 9.1, 5.0 Hz, 1H, H-14), 8.00 (s, 2H, H-8, 10), 7.86 (s, 1H, H-4), 7.57 (d, *J* = 9.1 Hz, 1H, H-12), 7.27 (d, *J* = 5.0 Hz, 1H, H-13). **¹³C-NMR** (75 MHz, DMSO- *d₆*): δ 165.66 (C-1), 162.31 (C-9), 147.16 (C-7), 144.55 (C-4), 133.05 (C-11), 132.65 (C-6), 128.23 (C-14), 127.98 (C-5), 117.59 (C-13), 117.54 (C-10), 114.84 (C-12), 114.21 (C-8). **Anal. Calc.** for C₁₂H₈FN₃O₃S: C, 49.15%; H, 2.75%; N, 14.33%; S, 10.93%. **Found:** C, 49.29%; H, 2.82%; N, 14.21%; S, 10.92%. **HPLC-MS:** t_{R}: 8.25 min (ACE-Excel 3 C18-PFP column, gradient 15-85% H₂O:CH₃CN 0.1% formic acid), [M+H]⁺ = 294 m/z.

### Preparing and obtaining (E)-N'-(2-fluoro-5-nitrobenzylidene)furan-2-carbohydrazide (A8H2)

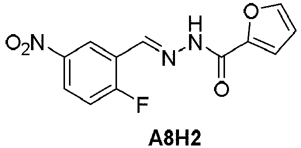

2-fluoro-5-nitrobenzaldehyde (240 mg, 1.44 mmol), 2-furoic acid hydrazide (150 mg, 1.2 mmol), MeOH (50 ml). It is purified by means of a Biotage system automated silica column chromatography using DCM/MeOH (9- 10%) obtaining an orange solid (0.26 g, 75%) (E > 99). **mp:** 187 - 188°C. **¹H-NMR** (300 MHz, DMSO- *d₆*): δ 12.23 (s, 1H, H-2), 8.68 (m, 2H, H-9, 10), 8.35 (m, 1H, H-14), 8.00 (m, 1H, H-4), 7.63 (m, 1H, H-12), 7.38 (s, 1H, H-7), 6.75 (m, 1H, H-13). **¹³C-NMR** (75 MHz, DMSO- *d₆*): δ 165.47 (C-1), 162.31 (C-6), 153.61 (C-9), 146.40 (C-4), 143.84 (C-10), 138.59 (C-8), 126.79 (C-7), 126.64 (C-14), 123.40 (C-5), 123.24 (C-13), 118.00 (C-10), 117.65 (C-12), 112.20 (C-8). **Anal. Calc.** for C₁₂H₈FN₃O₄: C, 51.99%; H, 2.91%; N, 15.16%. **Found:** C, 51.90%; H, 2.91%; N, 15.05%. **HPLC-MS:** t_{R}: 6.97 min (ACE-Excel 3 C18-PFP column, gradient 15-85% H₂O:CH₃CN 0.1% formic acid), [M+H]⁺ = 278 m/z.

### Preparing and obtaining (E)-(N'-(2-fluoro-5-nitrobenzylidene)-2-hydroxy-2-phenylacetohydrazide (A8H8)

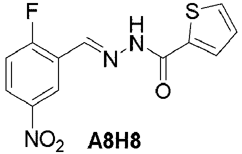

2-fluoro-5-nitrobenzaldehyde (220 mg, 1.3 mmol), mandelic acid hydrazide (150 mg, 1.1 mmol), MeOH (50 ml). It is purified by means of a Biotage system automated silica column chromatography using DCM/MeOH (0 - 4%) obtaining a white solid (0.16 g, 50%) (E > 99). **mp:** 237 - 238°C. **¹H-NMR** (300 MHz, DMSO- *d₆*): δ 12.20 (s, 1H, H-2),8.82 (s, 1H, H-10), 8.69 (s, 1H, H-8), 8.35 (m, 1H, H-14), 8.06 (s, 1H, H-7), 7.96 (s, 1H, H-4), 7.64 (m, 1H, H-12), 7.27 (m, 1H, H-13). **¹³C-NMR** (75 MHz, DMSO- *d₆*): δ 165.57 (C-1), 163.35 (C-6), 161.49 (C-9), 144.43 (C-4), 144.27 (2C, C-7, 14), 132.64 (C-11), 126.76 (2C, C-8, 10), 126.6 (C-5), 118.06 (C-13), 117.78 (C-12). **Anal. Calc.** for C₁₂H₈FN₃O₃S: C, 49.15%; H, 2.75%; N, 14.33%; S, 10.93%. **Found:** C, 49.36%; H, 2.79%; N, 14.24%; S, 10.95%. **HPLC-MS:** t_{R}: 7.85 min (ACE-Excel 3 C18-PFP column, gradient 15-85% H₂O:CH₃CN 0.1% formic acid), [M+H]⁺ = 318 m/z.

### Preparing and obtaining (Z)-N'-(4-chloro-3-nitrobenzylidene)furan-2-carbohydrazide (A5H2):

4-chloro-3-nitrobenzaldehyde (0.53 g, 2.9 mmol), 2-furoic acid hydrazide (0.32 g, 2.5 mmol), MeOH (50 ml). The solid is washed and filtered obtaining a white compound (0.33 g, 57%) (Z > 99%). mp: 191-192°C. **¹H-NMR** (300 MHz, DMSO-d6): δ 12.17 (s, 1H), 8.50 (s, 1H), 8.38 (m, 1H), 8.13 - 7.96 (m, 2H), 7.87 (m, 1H), 7.37 (s, 1H), 6.74 (m, 1H).13C-NMR (75 MHz, DMSO-d6): δ 153.0, 147.9, 146.2, 145.0, 134.9, 132.2 (2C), 131.5, 125.7, 123.5 (2C), 115.5, 112.2. Anal. Calc. for C₁₂H₈ClN₃O₄: C, 49.08%; H, 2.75%; N, 12.07%. Found: C, 49.16%; H, 2.89%; N, 12.17%. HPLC-MS: t_{R}: 7.74 min, [M+H]+ = 294 m/z.

### Example 2. In vitro NCS1/Ric8a interaction modulation assays

In order to evaluate the capacity of the compounds of the invention, A3H3, A5H2, and A3H4, for modulating interaction between human NCS-1 and Ric8as, HEK293 cells were transfected with a version of epitope V5-labeled Ric8a (Ric8a-V5) and NCS-1, performing cell incubation and then cell lysis with the product to be assayed. The amount of NCS-1-bound Ric8a-V5 was then assessed by means of the immunoprecipitation technique. The data (Figure 1) indicates that the presence of the compounds allows interaction with Ric8-a and demonstrated that the presence of product A3H3 increases the binding of NCS-1 with Ric8a by more than two fold.

### Example 3. Measuring the affinity of the compounds for the NCS-1 protein

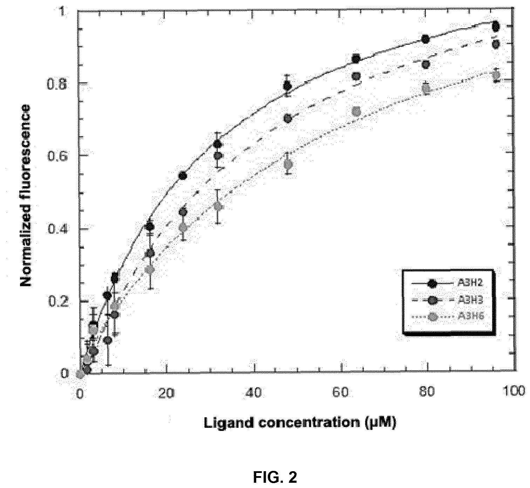

The affinity of the compounds for *d*NCS-1 was measured by means of the fluorescence spectroscopy technique. The emission intensity of the complex is analyzed, where the signal is monitored at increasing concentrations of ligand (1:0 to 1:30 equivalents), keeping the protein concentration 7.8 µL, 30.8 µM, 1 eq.) constant. The affinity constant is thereby obtained through the apparent disassociation constant. Figure 2 shows as an example the graph of A3H3, A3H2, and A3H6. The experiments are performed at 5°C with dNCS-1 in 50 mM Tris buffer, pH 7.9, 125 mM NaCl, 0.5 mM CaCl₂ with 5% DMSO in a total volume of 300 µL. The experiment is performed at the excitation wavelength of 295 nm and at the emission wavelength of 345 nm (maximum emission) depending on the compound in a Jobin Yvon Floromax4 spectrofluorometer equipped with a Peltier Thermostat; the obtained values are the result of the normalized average of the three repetitions for each measurement. The studied compounds do not exhibit emission in the measured range of fluorescence. The affinity constants found for the tested compounds are as follows:

| **Compound** | **K_{d} (µM)** |
|---|---|
| A3H2 | 32 ± 2 |
| A3H3 | 43 ± 6 |
| A3H6 | 61 ± 9 |
| A5H2 | 3.2 ± 0.3 |
| A5H6 | 3.2 ± 0.3 |
| A5H8 | 19 ± 8 |
| A7H2 | 25 ± 4 |
| A7H8 | 4 ± 1 |
| A8H2 | 7 ± 1 |
| A8H8 | 14 ± 2 |

### Clauses

1. A compound suitable for promoting NCS-1 and Ric8a interaction, wherein said compound comprises the structure of formula (I): 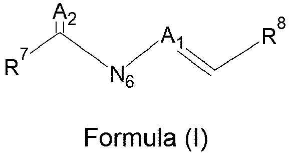 wherein A1 is nitrogen, N6 is NH, and A2 is oxygen;
   wherein A1 is at a distance from A2 of 2.63 ± 0.25 Å, wherein A1 is at a distance from N6 of 1.33 ± 0.15 Å, wherein A2 is at a distance from N6 of 2.32 ± 0.25 Å, wherein A1 is at a distance from R7 of 5.58 ± 0.55 Å, wherein A1 is at a distance from R8 of 3.77 ± 0.35 Å, wherein A2 is at a distance from R7 of 3.87 ± 0.35 Å, wherein A2 is at a distance from R8 of 6.04 ± 0.50 Å, wherein N6 is at a distance from R7 of 4.61 ± 0.45 Å, and wherein N6 is at a distance from R8 of 4.98 ± 0.35 Å;
   wherein the distance represents distances between the centers of the respective atoms;
   wherein R7 must interact with the N-terminus part of the hydrophobic cleft of the NCS1 protein (PDB ID code 4BY4), on one side with W30, F85, and L89, and on the other side with I51 and F55, through which π-π type hydrophobic interactions must be established, and comprises the following chemical structure selected from the list consisting of:
   - a heteroaryl with a single ring, preferably a thiophene or a furan;
   - a heteroaryl with two fused rings, preferably an indole group; or
   - a -[CH2]ₙ-R6 group, wherein n has any of the following values: 0, 1, or 2; and wherein R6 is a heterocycle with 2 fused rings or a single heterocyclic ring; and
   wherein R8 interacts with the region of the NCS1 protein (PDB ID code 4BY4) defined by amino acids V68, Y52, F72, F48, W103, and T92, with which π-π type hydrophobic interactions are established, and comprises the structure of formula II: 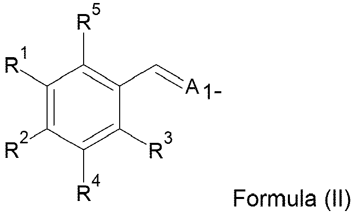 wherein at least one of the R¹ to R⁵ radicals of the structure of formula (II) is a nitro group and one or more of the R¹ to R⁵ radicals is selected from the list consisting of: H, F, Cl, Br, CF₃, or a hydroxyl group; and wherein the compound of formula (II) binds to the rest of the compound of formula (I) through the methine or methylidene group which in turn binds to group A1;
   or any of the isomers or salts thereof, for use in promoting interaction between NCS-1 and Ric8a proteins involved in the process of regulating the number of synapses in those diseases presenting with a reduction in the number of neuron synapses.
2. The compound for use according to clause 1, wherein one of the R¹ to R⁵ radicals of the structure of formula (II) is a nitro group, another one of the R¹ to R⁵ radical is selected from the list consisting of F, Cl, Br, CF₃, or a hydroxyl group, and the rest of the radicals are hydrogen.
3. The compound for use according to clause 1 or 2, wherein R7 is selected from the list consisting of:
   a. a thiophene or a furan; or
   b. an indole group.
4. The compound for use according to any of clauses 1 to 3, wherein said diseases presenting with a reduction in the number of neuron synapses are selected from the list consisting of Alzheimer's disease, Parkinson's disease, Huntington's disease, and other neurodegenerative diseases, as well as Angelman syndrome, fragile X chromosome syndrome, Rett syndrome, syndromes of the autism spectrum, epilepsy, Rett syndrome, schizophrenia, bipolar disorder, amyotrophic lateral sclerosis, Friedreich ataxia, progressive supranuclear palsy, frontotemporal dementia, Lewy body dementia, Creutzfeldt-Jakob disease.
5. The compound for use according to any of clauses 1 to 4, wherein the disease presenting with the reduction in the number of neuron synapses is Alzheimer's disease.
6. The compound for use according to any of clauses 1 to 5, wherein R¹ is OH.
7. The compound for use according to clause 6, wherein R² is NO₂.
8. The compound for use according to clause 7, wherein R³, R⁴, and R⁵ are H.
9. The compound for use according to any of clauses 1 to 5, wherein R₁ is NO₂.
10. The compound for use according to clause 9, wherein R₃ is F, Cl, or Br.
11. The compound for use according to clause 10, wherein R₃ is fluorine.
12. The compound for use according to any of clauses 9 to 11, wherein R², R⁴, and R⁵ are H.
13. The compound for use according to any of clauses 1 to 5, wherein R² is NO₂ and optionally R⁴ is OH.
14. The compound for use according to any of clauses 1 to 5, wherein R³ is NO₂ and optionally R¹ is F, Cl, or Br, preferably fluorine.
15. The compound for use according to any of clauses 1 to 5, wherein R⁴ is NO₂ and optionally R² or R³ is F, Cl, or Br, preferably chlorine.
16. The compound for use according to any of clauses 1 to 5, wherein R¹ is NO₂ and optionally R⁴ is F, Cl, or Br, preferably fluorine.
17. The compound for use according to any of clauses 1 to 5, wherein R¹ is NO₂ and optionally R⁴ is a hydroxyl.
18. The compound for use according to any of clauses 1 to 17, wherein R7 is a heteroaryl which is selected from thiophenyl or furanyl, wherein said groups can be optionally substituted with halogen.
19. The compound of formula (I) for use according to clause 1 which is selected from the following list:
   a. (*E*/*Z*)-*N*'-(3-hydroxy-4-nitrobenzylidene)-2-(1H-indol-3-yl)acetohydrazide (A3H3)
   b. (±)-(*E*/*Z*)-2-hydroxy-/V-(3-hydroxy-4-nitrobenzylidene)-2-phenylacetohydrazide (A3H6)
   c. (*E*)-4-(*tert*-butyl)-*N'*-(3-hydroxy-4-nitrobenzylidene)benzohydrazide (A3H17)
   d. (*Z*)-5-chloro-*N'*-(3-hydroxy-4-nitrobenzylidene)thiophene-2-carbohydrazide (A3H18)
   e. (*E*/*Z*)-3-hydroxy-*N*'-(3-hydroxy-4-nitrobenzylidene)-2-naphthohydrazide (A3H19)
   f. (*Z*)-*N*'-(4-chloro-3-nitrobenzylidene)furan-2-carbohydrazide (A5H2)
   g. (*E*/*Z*)-*N*'-(4-chloro-3-nitrobenzylidene)thiophene-2-carbohydrazide (A5H8)
   h. (*E*)-*N*'-(5-fluoro-2-nitrobenzylidene)furan-2-carbohydrazide (A7H2)
   i. (*E*/*Z*)-*N*'-(5-fluoro-2-nitrobenzylidene)thiophene-2-carbohydrazide (A7H8)
   j. (*E*)-*N*'-(2-fluoro-5-nitrobenzylidene)furan-2-carbohydrazide (A8H2)
   k. (*E*)-(*N*'-(2-fluoro-5-nitrobenzylidene)-2-hydroxy-2-phenylacetohydrazide (A8H8)
20. The compound of formula (I) which is selected from the following list:
   a. (*E*/*Z*)-*N*'-(3-hydroxy-4-nitrobenzylidene)-2-(1H-indol-3-yl)acetohydrazide (A3H3)
   b. (*E*)-4-(*tert*-butyl)-*N'*-(3-hydroxy-4-nitrobenzylidene)benzohydrazide (A3H17)
   c. (*Z*)-5-chloro-*N'*-(3-hydroxy-4-nitrobenzylidene)thiophene-2-carbohydrazide (A3H18)
   d. (*E*/*Z*)-3-hydroxy-*N*'-(3-hydroxy-4-nitrobenzylidene)-2-naphthohydrazide (A3H19)
   e. (*E*)-*N*'-(5-fluoro-2-nitrobenzylidene)furan-2-carbohydrazide (A7H2)
   f. (*E*/*Z*)-*N*'-(5-fluoro-2-nitrobenzylidene)thiophene-2-carbohydrazide (A7H8)
   g. (*E*)-*N*'-(2-fluoro-5-nitrobenzylidene)furan-2-carbohydrazide (A8H2)
   h. (*E*)-(*N*'-(2-fluoro-5-nitrobenzylidene)-2-hydroxy-2-phenylacetohydrazide (A8H8)
21. The compound according to claim 20 for use as a medicinal product.
22. A pharmaceutical composition comprising at least one compound according to clause 20 and at least one pharmaceutically acceptable vehicle.

## Claims

1. A compound suitable for promoting NCS-1 and Ric8a interaction, wherein said compound comprises the structure of formula (I): 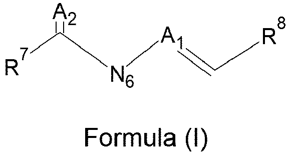 wherein A1 is nitrogen, N6 is NH, and A2 is oxygen;
wherein R7 comprises the following chemical structure selected from the list consisting of:
- a heteroaryl with a single ring, preferably a thiophene or a furan;
- a heteroaryl with two fused rings, preferably an indole group; or
- a -[CH2]ₙ-R6 group, wherein n has any of the following values: 0, 1, or 2; and wherein R6 is a heterocycle with 2 fused rings or a single heterocyclic ring; and
wherein R8 comprises the structure of formula II: 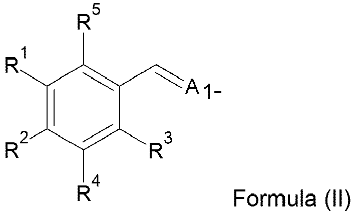 wherein at least one of the R¹ to R⁵ radicals of the structure of formula (II) is a nitro group and at least one or more of the R¹ to R⁵ radicals is selected from the list consisting of: F, Cl, Br, CF₃, or a hydroxyl group; and wherein the compound of formula (II) binds to the rest of the compound of formula (I) through the methine or methylidene group which in turn binds to group A1;
or any of the isomers or salts thereof,
for use in promoting interaction between NCS-1 and Ric8a proteins involved in the process of regulating the number of synapses in those diseases presenting with a reduction in the number of neuron synapses selected from the list consisting of: Alzheimer's disease, Parkinson's disease, Huntington's disease, Angelman syndrome, amyotrophic lateral sclerosis, Friedreich ataxia, progressive supranuclear palsy, frontotemporal dementia, Lewy body dementia, Creutzfeldt-Jakob disease; with the proviso that the compound of formula (I) is not the following compound:

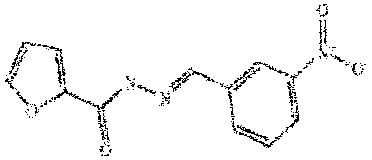

2. The compound of formula (I) for use according to claim 1, wherein A1 is at a distance from A2 of 2.63 ± 0.25 Å, wherein A1 is at a distance from N6 of 1.33 ± 0.15 Å, wherein A2 is at a distance from N6 of 2.32 ± 0.25 Å, wherein A1 is at a distance from R7 of 5.58 ± 0.55 Å, wherein A1 is at a distance from R8 of 3.77 ± 0.35 Å, wherein A2 is at a distance from R7 of 3.87 ± 0.35 Å, wherein A2 is at a distance from R8 of 6.04 ± 0.50 Å, wherein N6 is at a distance from R7 of 4.61 ± 0.45 Å, and wherein N6 is at a distance from R8 of 4.98 ± 0.35 Å;
wherein the distance represents distances between the centers of the respective atoms;
wherein R7 must interact with the N-terminus part of the hydrophobic cleft of the NCS1 protein (PDB ID code 4BY4), on one side with W30, F85, and L89, and on the other side with I51 and F55, through which π-π type hydrophobic interactions must be established ;
and wherein R8 interacts with the region of the NCS1 protein (PDB ID code 4BY4) defined by amino acids V68, Y52, F72, F48, W103, and T92, with which π-π type hydrophobic interactions are established.

3. The compound of formula (I) for use according to claim 1 or 2, wherein R¹ is selected from OH, NO₂, F, Cl, or Br, and wherein R² to R³ are selected from the list consisting of NO₂, F, Cl, or Br.

4. The compound for use according to any of claims 1 to 3, wherein R₁ is OH.

5. The compound for use according to claim 4, wherein R₂ is NO₂.

6. The compound for use according to claim 5, wherein R₃, R₄, and R₅ are H.

7. The compound for use according to any of claims 1 to 3, wherein R₁ is NO₂.

8. The compound for use according to claim 7, wherein R₂ is F, Cl, or Br.

9. The compound for use according to claim 8, wherein R₂ is chlorine.

10. The compound for use according to any of claims 8 or 9, wherein R₃, R₄, and R₅ are H.

11. The compound for use according to claim 7, wherein R₂ is H.

12. The compound for use according to claim 11, wherein R₃ is F, Cl, or Br.

13. The compound for use according to claim 12, wherein R₃ is fluorine.

14. The compound for use according to any of claims 1 to 3, wherein R₁ is F, Cl, or Br.

15. The compound for use according to claim 14, wherein R₁ is fluorine.

16. The compound for use according to any of claims 14 or 15, wherein R₂, R₄, and R₅ are H.

17. The compound for use according to claim 16, wherein R₃ is NO₂.

18. The compound for use according to any of claims 1 to 17, wherein R₇ is a heteroaryl which is selected from thiophenyl or furanyl.

19. The compound of formula (I) for use according to any of claims 1 to 3, which is selected from the following list:
a. (*E*/*Z*)-*N*'-(3-hydroxy-4-nitrobenzylidene)-2-(1H-indol-3-yl)acetohydrazide (A3H3)
b. (±)-(*E*/*Z*)-2-hydroxy-*N*'-(3-hydroxy-4-nitrobenzylidene)-2-phenylacetohydrazide (A3H6)
c. (*E*)-4-(*tert*-butyl)-*N'*-(3-hydroxy-4-nitrobenzylidene)benzohydrazide (A3H17)
d. (*Z*)-5-chloro-*N'*-(3-hydroxy-4-nitrobenzylidene)thiophene-2-carbohydrazide (A3H18)
e. (*E*/*Z*)-3-hydroxy-*N*'-(3-hydroxy-4-nitrobenzylidene)-2-naphthohydrazide (A3H19)
f. (*Z*)-*N*'-(4-chloro-3-nitrobenzylidene)furan-2-carbohydrazide (A5H2)
g. (*E*/*Z*)-*N*'-(4-chloro-3-nitrobenzylidene)thiophene-2-carbohydrazide (A5H8)
h. (*E*)-*N*'-(5-fluoro-2-nitrobenzylidene)furan-2-carbohydrazide (A7H2)
i. (*E*/*Z*)-*N*'-(5-fluoro-2-nitrobenzylidene)thiophene-2-carbohydrazide (A7H8)
j. (*E*)-*N*'-(2-fluoro-5-nitrobenzylidene)furan-2-carbohydrazide (A8H2)
k. (*E*)-(*N*'-(2-fluoro-5-nitrobenzylidene)-2-hydroxy-2-phenylacetohydrazide (A8H8).

20. The compound of formula (I) which is selected from the following list:
a. (*E*/*Z*)-*N*'-(3-hydroxy-4-nitrobenzylidene)-2-(1H-indol-3-yl)acetohydrazide (A3H3)
b. (*E*)-4-(*tert*-butyl)-*N'*-(3-hydroxy-4-nitrobenzylidene)benzohydrazide (A3H17)
c. (Z)-5-chloro-*N*'-(3-hydroxy-4-nitrobenzylidene)thiophene-2-carbohydrazide (A3H18)
d. (*E*/*Z*)-3-hydroxy-*N*'-(3-hydroxy-4-nitrobenzylidene)-2-naphthohydrazide (A3H19)
e. (*E*)-*N*'-(5-fluoro-2-nitrobenzylidene)furan-2-carbohydrazide (A7H2)
f. (*E*/*Z*)-*N*-(5-fluoro-2-nitrobenzylidene)thiophene-2-carbohydrazide (A7H8)
g. (*E*)-*N*'-(2-fluoro-5-nitrobenzylidene)furan-2-carbohydrazide (A8H2)
h. (*E*)-(*N*'-(2-fluoro-5-nitrobenzylidene)-2-hydroxy-2-phenylacetohydrazide (A8H8).

21. The compound according to claim 20 for use as a medicinal product.

22. A pharmaceutical composition comprising at least one compound according to claim 20 and at least one pharmaceutically acceptable vehicle.
